# EUROPEAN PATENT APPLICATION

(11) **EP 4 552 510 A1**
(43) Date of publication of application: **14.05.2025**
(21) Application number: 23209386.4
(22) Date of filing: 13.11.2023
(51) Int. Cl.: A23L 33/105, A23P 10/22, A61K 9/20, A61K 36/185, A61K 36/484, A61K 36/82, A61K 45/06, A61P 3/04

(54) **DIETARY SUPPLEMENT FOR COMPREHENSIVE WEIGHT LOSS SUPPORT**

(71) Applicant: Mybestpharm Spolka Akcyjna, 47-220 Kedzierzyn-Kozle (PL)
(72) Inventor: GRZYBOWSKI, Mariusz, Warszawa (PL)
(74) Representative: AOMB Polska Sp. z.o.o.

(57) **Abstract**

The invention relates to a dietary supplement that comprehensively supports the weight loss process, containing a mixture of active substances of plant origin, vitamins, and minerals. The selected components of the blend have a positive impact on energy metabolism, carbohydrate metabolism, fat metabolism, regulate the concentration of sugars and fats in the bloodstream, contribute to appetite control, and promote the utilization of fats for energy processes during physical activity.

## Description

### Field of the invention

The invention is related to a dietary supplement comprehensively supporting the weight loss process, said supplement containing a set of active ingredients of plant origin, vitamins, and minerals.

### Background art

There are many dietary supplements known for supporting the weight loss process in individuals aiming to reduce the amount of body fat for both aesthetic and health reasons, especially in overweight individuals. Typically, dietary supplements contain a combination of several active ingredients.

For example, in the patent specification PL 209905 B1 a preparation for treating obesity and associated metabolic syndrome was disclosed, containing 20-90% by weight of green tea extract, 2-30% by weight of *Coleus Forskohlii* extract, 5-58% by weight of *Yerba maté* extract (Paraguayan holly), and 7.5-45% by weight of Betula alba extract.

EP 1640015 A2 pertains to drugs and dietary food containing cinnamon or cinnamon extract and their use for reducing energy absorption from food, especially in the prevention and treatment of obesity and hyperglycemia.

CN 104138002 A discloses a formulation containing green coffee bean extract, *Citrus aurantium* (bitter orange) extract, lotus leaf extract, bitter melon extract, spirulina extract, *Ginkgo biloba* (Japanese maidenhair tree) extract.

US 11052125B2 pertains to a weight loss-promoting composition for mammals, containing extracts of *Cyperus rotundus, Garcinia sp., Coleus forskohlii* (forskolin). The composition may also optionally contain black pepper fruit extract.

US 20080268075 A1 discloses a composition of herbal extracts suitable for weight control, containing a mixture of *Garcinia* extract, green tea extract, green coffee extract, and banaba extract.

The dietary supplement named "My Best Slim" (from the company MyBestPharm Sp. z o.o.) included a combination of 15 active ingredients: chromium, extract of Paraguayan holly leaves (*Ilex paraguarensis*), also known as *Yerba mate,* bitter orange extract, green coffee bean extract, selenium, cayenne pepper extract, black pepper fruit extract, grape seed extract, guarana extract, Garcinia cambogia fruit extract containing 60% hydroxycitric acid (HCA), bladderwrack extract, vitamin B2, vitamin B12, gymnema leaf extract (*Gymnema sylvestre*)*,* and L-carnitine tartrate.

On the other hand, the dietary supplement named "MyBestSlim 2.0" (from the company MyBestPharm Sp. z o.o.) contained a combination of up to 19 active ingredients: chromium picolinate, extract of Paraguayan holly leaves, bitter orange extract, green coffee bean extract, gymnema leaf extract, cayenne pepper extract, black pepper fruit extract, grape seed extract, guarana extract, Indian nettle extract, Chinese licorice root extract, green tea leaf extract, horsetail herb extract, Ceylon cinnamon bark extract, selenium yeast, cyanocobalamin, L-carnitine tartrate, vitamin B2, and bladderwrack extract.

The specific qualitative and quantitative selection of the mixture of active ingredients in a supplement is influenced by various decisions, including the availability of a given ingredient, its biological properties, metabolic pathway, potential risks associated with adverse interactions with other mixture components, and the presence of harmful contaminants.

The vast variety of commercially available dietary supplements supporting weight loss and fat tissue reduction reflects the diversity of approaches to the weight loss process or its individual metabolic aspects, the individualization of patient needs (including both healthy individuals and those suffering from cardiovascular diseases, diabetes, cancer, etc.), as well as the availability and cost of used ingredients. Despite the large number of different weight loss supplements, there is still a demand for new, alternative combinations of known active ingredients, allowing for increased effectiveness of fat tissue reduction, enhanced sustainability of the effects of such a process, and providing the most comprehensive approach to this issue, consistent with general recommendations for a healthy lifestyle, nutrition, and physical activity.

### Summary of the invention

The aim of the invention was to provide a dietary supplement in the form of a single preparation, enabling comprehensive support for the weight loss process, free from active ingredients burdened with the risk of harmful contaminants, and conducive to patients adhering to the recommended dosage regimen.

The subject of the invention is a dietary supplement comprehensively supporting the weight loss process, which, as active ingredients, includes chromium, selenium, vitamin B2, vitamin B12, extract from the leaves of Paraguayan holly (*Ilex paraguarensis*), extract from bitter orange (*Citrus Aurantium*)*,* extract from green coffee beans (*Coffea arabica*)*,* extract from cayenne pepper (*Capsicum annuum*)*,* also known as annual pepper, extract from black pepper fruits (*Piper nigrum*)*,* grape seed extract (*Vitis vinifera L.*)*,* guarana seed extract (*Paullinia cupana Kunth*)*,* bladderwrack extract (*Fucus vesiculosus*)*,* gymnema leaf extract (*Gymnema sylvestre*)*,* L-carnitine tartrate, extract from Indian nettle (*Coleus Forskohlii*)*,* extract from licorice root (*Glycyrrhiza glabra L.*)*,* green tea leaf extract (*Camellia sinensis*)*,* horsetail herb extract (*Equisetum arvense*)*,* and cinnamon bark extract (*Cinnamomum cassia*)*.* It is characterized by additionally containing at least one additional active ingredient selected from spirulina, extract from Damian leaves (*Turnera diffusa L.*)*,* extract from the fruit of Malabar tamarind (*Garcinia Cambogia*)*,* extract from African mango seeds (*Irvingia gabonensis L.*)*,* extract from chicory root (*Cichorium intybus L.*)*,* extract from black elderberry flowers (*Sambucus nigra L.*)*,* extract of ground elder leaves (*Aegopodium podagraria L.*)*,* extract from Shilajit Mumio (*Asphaltum punjabianum*)*,* and extract from leaves of ash tree (*Fraxinus Excelsior*)*.*

Preferably, the diet supplement according to the invention comprises spirulina, extract of Damiana leaves (*Turnera diffusa L.*)*,* extract of Malabar tamarind fruit (*Garcinia Cambogia*)*,* extract of African mango seeds (*Irvingia gabonensis L.*)*,* extract of chicory root (*Cichorium intybus L.*)*,* extract of black elderflower (*Sambucus nigra L.*)*,* extract of ground elder leaves (*Aegopodium podagraria L.*)*,* Shilajit Mumio extract (*Asphaltum punjabianum*)*,* and extract of ash tree leaves (*Fraxinus Excelsior*)*,* as well as magnesium stearate.

Preferably, in the dietary supplement according to the invention, chromium is in the form of chromium picolinate, selenium is in the form of selenium yeast, vitamin B12 is in the form of methylcobalamin, bitter orange fruit extract contains 6% by weight of synephrine, green coffee bean extract contains 50% by weight of chlorogenic acid, gymnema leaf extract contains 25% by weight of gymnemic acid, cayenne pepper fruit extract contains 2% by weight of capsaicin, black pepper fruit extract contains 95% by weight of piperine, grape seed extract contains 95% by weight of proanthocyanidins, guarana seed extract contains 22% by weight of caffeine, Indian nettle extract contains 10% by weight of forskolin, licorice root extract contains 1% by weight of glycyrrhizic acid, green tea leaf extract contains 40% by weight of epigallocatechin gallate (EGCG), bladderwrack extract contains 0.1% by weight of iodine, Damiana leaf extract contains 1% by weight of beta-sitosterol, and Malabar tamarind fruit extract contains 60% by weight of hydroxycitric acid.

Preferably, the dietary supplement according to the invention is in the form of capsules.

In a particularly preferred embodiment, the dietary supplement according to the invention has the following composition of active ingredients per single capsule:
0.137 parts by weight of chromium picolinate,
31.667 parts by weight of Paraguayan holly leaf extract,
33.333 parts by weight of bitter orange fruit extract,
16.667 parts by weight of extract of green coffee beans,
50.000 parts by weight of gymnema leaf extract,
13.333 parts by weight of cayenne pepper fruit extract,
0.281 parts by weight of black pepper fruit extract,
15.000 parts by weight of grape seed extract,
68.182 parts by weight of guarana seed extract,
25.000 parts by weight of Indian stinging nettle extract,
16.667 parts by weight of licorice root extract,
66.667 parts by weight green tea leaf extract,
17.333 parts by weight of horsetail herb extract,
5.000 parts by weight of extract of the bark of Ceylon cinnamon,
5.000 parts by weight of selenium-enriched yeast,
0.042 parts by weight of methylcobalamin,
101.500 parts by weight of L-carnitine tartrate,
1.000 parts by weight of vitamin B2 (riboflavin),
16.667 parts by weight of bladderwrack extract,
10.000 parts by weight of spirulin,
20.000 parts by weight of Damiana leaf extract,
133.333 parts by weight of Garcinia cambogia fruit extract,
4.167 parts by weight of African mango seed extract,
8.003 parts by weight of chicory root extract,
15.000 parts by weight of black elderberry flower extract,
0.250 parts by weight of ground elder leaves extract,
8.333 parts by weight of Shilajit Mumio extract, and
3.333 parts by weight of ash tree leaves extract.

By utilizing a blend of 28 active plant-based ingredients, minerals, and vitamins, a comprehensive dietary supplement supporting the weight loss process has been provided. This supplement addresses various aspects of human body functioning, influencing the undesirable accumulation of body fat or, conversely, facilitating fat reduction through the modification of the dynamics and intensity of metabolic processes. The qualitative composition of the mixture of active ingredients in the invention allows for the optimization of achieved effects while maintaining a simple dosage scheme. Instead of taking several different preparations in the form of separate oral dosages, patients can use a single dietary supplement comprehensively, implementing a plan to reduce body fat mass, effectively complemented by changes in diet and lifestyle. This directly translates into better patient compliance with therapeutic recommendations (patient compliance, patient adherence), as it is easier for them to follow the dosage regimen of a single preparation compared to a series of different preparations. This, in turn, contributes to the greater effectiveness of the entire weight loss process.

The ingredients of the dietary supplement according to the invention exhibit effects on multiple levels related to supporting the weight loss process:
- positively affect energy metabolism,
- positively affect carbohydrate metabolism,
- positively affect fat metabolism,
- regulate the concentration of glucose and fats in the blood serum,
- contribute to appetite suppression,
- promote the utilization of fats for energy processes during physical activity.

Owing to the multi-faceted action of individual active ingredients, the supplement provides comprehensive support for the weight loss process through a single preparation.

The product is in the form of capsules, containing a beneficial mixture of active ingredients along with excipients, including anti-caking agents (e.g., magnesium stearate). The capsule shells are advantageously made from cellulose derivatives, such as hydroxypropyl methylcellulose. The absence of animal-derived ingredients in the capsules of the dietary supplement according to the invention (e.g., gelatin capsules) makes the product suitable for vegetarians and vegans.

### Detailed description of the invention

As mentioned above, the subject of the invention is a dietary supplement consisting of a combination of 28 different active ingredients, enabling comprehensive support for the weight loss process.

The first and broadest category of active ingredients used in the dietary supplement according to the invention consists of plant-derived materials.

Extract from the leaves of yerba mate (*Ilex paraguariensis*) contains caffeine as well as vitamins A, B1, B2, C, minerals, inositol, choline, nicotinic acid, and flavonoids. It has stimulating effects, reduces the progression of atherosclerosis, promotes the body's detoxification from heavy metals and toxins, dilates blood vessels, and supports weight loss processes.

Extract from bitter orange contains synephrine, which promotes the breakdown of fats and supports digestion.

Green coffee bean extract contains chlorogenic acid, which reduces the absorption and utilization of carbohydrates (primarily glucose) from food. In the longer term, this may contribute to weight and fat mass reduction.

Gymnema leaf extract contains gymnemic acid, which promotes maintaining a healthy body weight and helps control appetite by contributing to the maintenance of normal blood sugar levels. Additionally, it has a positive impact on the metabolism of fats and sugars.

Extract from cayenne pepper (Cayenne) contains capsaicin, which influences fat metabolism, aiding in the control of body weight.

Extract from black pepper contains piperine, supporting digestive processes.

Grape seed extract contains proanthocyanidins, which belong to antioxidants widely recognized as health-promoting substances.

Guarana extract contains caffeine, which has a beneficial effect on fat metabolism.

Indian nettle leaf extract (*Coleus barbatus, Plectranthus barbatus,* and - though commonly but incorrectly - *Coleus forskalaei*/*forskohili*) contains forskolin, which increases the concentration of cyclic AMP (cAMP) in cells, contributing to the maintenance of a healthy body weight and regulation of fat metabolism.

Licorice root extract (*Glycyrrhiza glabra L.*) contains 180-glycyrrhizic acid (also known as glycyrrhetic acid, 18β-glycyrrhetinic acid), which contributes to balanced metabolism and weight loss.

Green tea leaf extract contains catechins, particularly epigallocatechin gallate (EGCG). Catechins play a role in maintaining or achieving a healthy body weight, support the proper functioning of the large intestine, help maintain normal levels of glucose and cholesterol in the blood, and protect DNA from oxidative damage. Due to their properties, catechins have a beneficial effect on maintaining normal blood pressure, especially in individuals with arterial hypertension.

Field horsetail herb extract, in addition to its anti-cancer, anti-hemorrhagic, and anti-inflammatory properties, also supports metabolism, contributing to the prevention of the accumulation of adipose tissue.

Bark extract from Cinnamomum cassia (*Cassia cinnamon*) contains compounds that support maintaining the proper level of glucose in the blood, aid in the proper functioning of the stomach (helping to avoid bloating), exhibit significant antioxidant activity, and also influence overall invigoration (feeling of increased energy) and reduction of fatigue.

Bladderwrack extract (*Fucus Vesiculosus*) contains iodine, which supports the maintenance of a normal energy metabolism and plays a crucial role in ensuring the proper functioning of the thyroid gland.

Damiana leaf extract (*Turnera diffusa L.*) contains bioactive substances such as flavonoids (apigenin), cyanogenic glycosides (gonzalitozine, tetraphyllin B), phytosterols (especially beta-sitosterol), phenolic glycosides (arbutin), alkanes (tricosane), hydrocarbons (hexadecanol), essential oils, and damianin. Current in vitro and in vivo research results indicate that Damiana leaf extract may regulate, i.e., lower blood glucose levels, alleviate the effects of oxidative stress, reduce states of nervous tension, support the building and recovery of physical and mental resilience, help prevent photoaging of the skin, stimulate libido, and exert cytotoxic effects on cancer cells. Damiana leaf extract contributes to weight control by reducing appetite and promoting a feeling of fullness.

The extract from the fruit of the Malabar tamarind tree (*Garcinia Cambogia*) contains hydroxycitric acid (HCA), which is an inhibitor of ATP-citrate lyase, an enzyme involved in the synthesis of fatty acids. HCA inhibits lipogenesis, limiting the accumulation of fats in the body and contributing to the regulation of triglyceride and cholesterol levels in the blood. By accelerating and increasing the transport of fatty acids to the mitochondria, HCA reduces appetite. Additionally, HCA contributes to a slight increase in body temperature, promoting the burning of fat tissue and speeding up metabolism. Despite reports of possible negative effects, including liver damage, dry mouth, nausea, gastrointestinal discomfort, headaches, and potential adverse interactions with diabetes, asthma, and blood clotting medications, the use of this ingredient in the dietary supplement according to the invention has been deemed acceptable. This is particularly true as recent producers of the extract from Southeast Asia avoid using ethylene oxide for sterilizing plant material, minimizing the risk of contamination. It is essential to emphasize that the significant weight-loss effect of this ingredient is not only about beauty and aesthetics but, in many cases, leads to a substantial reduction in the risk of many serious and common diseases associated with overweight.

Extract from the seeds of the African mango (*Irvingia gabonensis L.*) contains fiber, which, when swelling, slows down the emptying of the stomach. This leads to a more gradual absorption of sugars from food, which, in turn, may inhibit the post-meal increase in blood sugar levels. The fiber from African mango seeds can bind with bile acids in the intestines, leading to their excretion from the body in feces. This process requires the body to convert a greater amount of cholesterol into bile acids, indirectly contributing to lowering cholesterol levels and other lipids in the blood.

Extract from the root of chicory (*Cichorium intybus L.*) is a rich source of inulin, with content reaching up to 40%. Inulin is a natural prebiotic, an undigested component in the digestive tract that serves as nourishment for intestinal bacteria, supporting their growth and development. In the digestive tract, inulin exhibits effects similar to dietary fiber, stimulating intestinal peristalsis. Additionally, chicory root extract has hypoglycemic and hypolipidemic properties. Thanks to the presence of chicoric acid, the extract demonstrates both insulin-sensitizing and insulin-secreting stimulating effects.

Extract from the flower of black elderberry (*Sambucus nigra L.*) has a circulatory stimulating and blood sugar-lowering effect. Additionally, due to its high antioxidant content, it strengthens the body's immunity in cases of infections. The presence of flavonoids and phytosterols in the elderflower extract supports metabolism.

Extract from the leaves of ground elder (*Aegopodium podagraria L.*)*,* traditionally used in alleviating gout, also has a positive impact on the digestive system, supporting digestion, and improving metabolism.

Extract from Shilajit Mumio (*Asphaltum punjabianum*) is a source of fulvic, humic, and humic acids, as well as triterpenes, sterols, and aromatic carboxylic acids. It exhibits various health-promoting properties, including support for memory and learning processes, antidiabetic, antioxidant, heart-stimulating, and hypolipidemic effects.

Extract from the leaves of common ash (*Fraxinus Excelsior*) is a source of coumarins, occurring both in free form and as glycosides. It also contains flavonoids, triterpenes, sterols, iridoids, mannitol, glycosides (such as syringin), phenolic acids, and various mineral compounds. Among its range of properties, it demonstrates diuretic and gently laxative effects, anti-inflammatory properties, stimulates liver function, and regulates cholesterol levels in the blood. Extract from common ash leaves is traditionally used as an additive to dietary supplements supporting weight loss/maintenance of a healthy body weight.

The second significant category of active ingredients in the dietary supplement according to the invention comprises minerals, specifically chromium and selenium. Chromium, preferably in the form of picolinate, supports the maintenance of a proper metabolism, contributing to the maintenance of a normal metabolism of macronutrients and helping to maintain a normal blood glucose level. Selenium, preferably in the form of selenium yeast, helps protect cells from oxidative stress and supports the proper functioning of the thyroid.

The third important group of active ingredients in the dietary supplement according to the invention comprises vitamins, specifically vitamin B2 (riboflavin) and vitamin B12. Vitamins B2 and B12 contribute to the maintenance of a normal energy metabolism. Unlike the synthetic form, cyanocobalamin, methylcobalamin is a form of vitamin B12 (cobalamin) naturally occurring in fish, meat, eggs, and milk. Although studies indicate that cyanocobalamin is somewhat more stable and slightly better absorbed than methylcobalamin (J.F. Adams, S.K. Ross, L. Mervyn, K. Boddy, P. King, Scand J Gastroenterol, 1971;6(3):249-52, doi: 10.3109/00365527109180702), other research suggests that cyanocobalamin is excreted to a much greater extent in the urine, indicating a greater ability of methylcobalamin retention in the body (K. Okuda, K. Yashima, T. Kitazaki, I. Takara, J Lab Clin Med., 1973 Apr; 81(4):557-67).

Methylcobalamin, as a form of vitamin B12, is particularly recommended in cases of negative symptoms in the nervous system and in conditions where the digestive system inadequately produces enzymes responsible for converting cyanocobalamin into methylcobalamin in the intestines. The use of methylcobalamin in the dietary supplement allows bypassing this significant obstacle to proper absorption of vitamin B12 into the blood, ensuring a more predictable and stable level of vitamin B12 supplementation than cyanocobalamin.

The dietary supplement according to the invention also contains L-carnitine in the form of a salt, which increases the level of fat energy utilization in physically active individuals.

### Examples

### Example 1:

The capsule contains a mixture of active ingredients along with a bulk-forming excipient.

**Table 1: Capsule Composition**

| **Active ingredients** | **Quantity of active ingredient (+/- 10%)** |
|---|---|
| Chromium picolinate | 0.137 mg, including 16.667 µg of chromium |
| Paraguayan holly leaf extract (*Ilexparaguarensis*) DER 10:1 | 31.667 mg |
| Bitter orange fruit extract (*Citrus Aurantium*) standardized to 6% synephrine by weight | 33.333 mg, including 2.000 mg of synephrine |
| Green coffee beans extrect (*Coffea arabica*) standardized to 50% weight of chlorogenic acid | 16.667 mg, including 8.330 mg of chlorogenic acid |
| Gymnema leaf extract (*Gymnema sylvestre*) standardized to 25% weight of gymnemic acid | 50.000 mg, including 12.500 mg of gymnemic acid |
| Cayenne pepper fruit extract (*Capsicum annuum*) standardized to 2% weight of capsaicin | 13.333 mg, including 0.267 mg of capsaicin |
| Black pepper fruit extract (*Piper nigrum*) standardized to 95% weight of piperine | 0.281 mg, including 0.267 mg of piperine |
| Grape seed extract (*Vitis vinifera L.*) standardized to 95% weight of proanthocyanidins | 15.000 mg, including 14.250 mg of proanthocyanidins |
| Guarana seed extract (*Paullinia cupana Kunth*) standardized to 22% weight of caffeine | 68.182 mg, including 15.000 mg of caffeine |
| Indian nettle extract (*Coleus Forskohlii*) standardized to 10% weight of forskolin | 25.000 mg, including 2.500 mg of forskolin |
| Licorice root extract (*Glycyrrhiza glabra L.*) standardized to 1% weight of glycyrrhizic acid | 16.667 mg, including 0.167 mg of glycyrrhizic acid |
| Green tea leaf extract (*Camellia sinensis*) standardized to 40% weight of EGCG (Epigallocatechin gallate) | 66.667 mg, including 26.667 mg of EGCG (Epigallocatechin gallate) |
| Horsetail herb extract (*Equisetum arvense L.*) DER 6:1 standardized to 7% silicon dioxide | 17.333 mg, including 1.213 mg of silicon dioxide |
| Extract of the bark of Ceylon cinnamon (*Cinnamomum cassia*) DAER 10:1 | 5.000 mg |
| Selenium-enriched yeast standardized to 0.2% selenium | 5.000 mg, including 10 µg of selenium |
| Methylcobalamin 1% - vitamin B12 (no additives) | 0.042 mg, including 0.417 µg of vitamin B12 |
| L-carnitine tartrate | 101.500 mg of L-carnitine tartrate |
| Vitamin B2 (riboflavin) (no additives) | 1.000 mg |
| Bladderwrack extract (*Fucus vesiculosus*) standardized to 0.1% weight of iodine | 16.667 mg, including 16.667 µg of iodine |
| Spirulina | 10.000 mg |
| Damiana leaf extract (*Turnera diffusa L.*) Standardized to 1% beta-sitosterols, DER 4:1 | 20.000 mg, including 0.2 mg of beta-sitosterols |
| Malabar tamarind fruit extract (*Garcinia Cambogia*) containing 60% hydroxycitric acid (HCA), DER 5:1 | 133.333 mg, including 80.000 mg of hydroxycitric acid |
| African mango seed extract (*Irvingia gabonensis L*.) DER 20:1 | 4.167 mg |
| Chicory root extract (*Cichorium intybus L.*) DER 20:1 | 8.003 mg |
| Black elderberry flower extract (*Sambucus nigra L*.) DER 20:1 | 15.000 mg |
| Extract of ground elder leaves (*Aegopodium podagraria L.*) DER 10: 1 | 0.250 mg |
| Shilajit Mumio extract (*Asphaltum punjabianum*) DER 10:1 | 8.333 mg |
| Ash tree leaves extract (*Fraxinus Excelsior*) DER 25:1 | 3.333 mg |
| | |

| **Excipients** | **Amount** |
|---|---|
| Magnesium stearate (anti-caking agent) | 7.500 mg |
| | |
| **Average capsule fill weight** | 693.390 mg |

| **Coating agents** | |
|---|---|
| Hydroxypropyl methylcellulose (HPMC) | |
| **Average capsule coating weight** | 123.000 mg |
| **Average total weight of the capsule** | 816.39 mg |

Recommended daily dose: adults - 6 capsules (3 capsules approximately 20 minutes before the first meal and 3 capsules approximately 20 minutes before the main meal).

More noticeable effects can be observed after the third month of use, although the timing of observable effects depends on the degree of overweight, metabolic efficiency, and other individual factors. The first effect (in some cases appearing as early as after 1 week of use) is a reduction in appetite, especially for sweets. In individuals particularly responsive to the supplement, the initial effects related to weight reduction can be observed after 2-3 weeks.

## Claims

1. A dietary supplement for comprehensive weight loss support, which, as active ingredients, includes chromium, selenium, vitamin B2, vitamin B12, extract from the leaves of Paraguayan holly (*Ilex paraguarensis*), extract from bitter orange (*Citrus Aurantium*)*,* extract from green coffee beans (*Coffea arabica*)*,* extract from cayenne pepper (*Capsicum annuum*)*,* extract from black pepper fruits (*Piper nigrum*)*,* grape seed extract (*Vitis vinifera L.*)*,* guarana seed extract (*Paullinia cupana Kunth*)*,* bladderwrack extract (*Fucus vesiculosus*)*,* gymnema leaf extract (*Gymnema sylvestre*)*,* L-carnitine tartrate, extract from Indian nettle (*Coleus Forskohlii*)*,* extract from licorice root (*Glycyrrhiza glabra L.*)*,* green tea leaf extract (*Camellia sinensis*)*,* horsetail herb extract (*Equisetum arvense*)*,* and cinnamon bark extract (*Cinnamomum cassia*)*,* **characterised in that** it additionally comprises at least one active ingredient selected from spirulina, extract from Damiana leaves (*Turnera diffusa L.*)*,* extract from the fruit of Malabar tamarind (*Garcinia Cambogia*)*,* extract from African mango seeds (*Irvingia gabonensis L.*)*,* extract from chicory root (*Cichorium intybus L.*)*,* extract from black elderberry flowers (*Sambucus nigra L.*)*,* extract of ground elder leaves (*Aegopodium podagraria L.*), extract from Shilajit Mumio (*Asphaltum punjabianum*)*,* and extract from leaves of ash tree (*Fraxinus Excelsior*)*.*

2. The dietary supplement according to claim 1, **characterised in that** it comprises spirulina, Damiana leaf extract (*Turnera diffusa L.*)*,* Malabar tamarind fruit extract (*Garcinia Cambogia*)*,* African mango seed extract (*Irvingia gabonensis L.*)*,* chicory root extract (*Cichorium intybus L.*)*,* black elderberry flower extract (*Sambucus nigra L.*)*,* ground elder leaf extract (*Aegopodium podagraria L.*)*,* Shilajit Mumio extract (*Asphaltum punjabianum*)*,* and ash tree leaf extract (*Fraxinus Excelsior*)*,* as well as magnesium stearate.

3. The dietary supplement according to claim 2, **characterised in that** chromium is in the form of chromium picolinate, selenium is in the form of selenium yeast, vitamin B12 is in the form of methylcobalamin, bitter orange fruit extract contains 6% by weight of synephrine, green coffee bean extract contains 50% by weight of chlorogenic acid, gymnema leaf extract contains 25% by weight of gymnemic acid, cayenne pepper fruit extract contains 2% by weight of capsaicin, black pepper fruit extract contains 95% by weight of piperine, grape seed extract contains 95% by weight of proanthocyanidins, guarana seed extract contains 22% by weight of caffeine, Indian nettle extract contains 10% by weight of forskolin, licorice root extract contains 1% by weight of glycyrrhizic acid, green tea leaf extract contains 40% by weight of epigallocatechin gallate (EGCG), bladderwrack extract contains 0.1% by weight of iodine, Damiana leaf extract contains 1% by weight of beta-sitosterol, and Malabar tamarind fruit extract contains 60% by weight of hydroxycitric acid.

4. The dietary supplement according to claim 3, **characterised in that** it is in the form of a capsule.

5. The dietary supplement according to claim 4, **characterised in that** it has the following composition of active substances per single capsule.
0.137 parts by weight of chromium picolinate,
31.667 parts by weight of Paraguayan holly leaf extract,
33.333 parts by weight of bitter orange fruit extract,
16.667 parts by weight of extract of green coffee beans,
50.000 parts by weight of gymnema leaf extract,
13.333 parts by weight of cayenne pepper fruit extract,
0.281 parts by weight of black pepper fruit extract,
15.000 parts by weight of grape seed extract,
68.182 parts by weight of guarana seed extract,
25.000 parts by weight of Indian nettle extract,
16.667 parts by weight of licorice root extract,
66.667 parts by weight green tea leaf extract,
17.333 parts by weight of horsetail herb extract,
5.000 parts by weight of extract of the bark of Ceylon cinnamon,
5.000 parts by weight of selenium-enriched yeast,
0.042 parts by weight of methylcobalamin,
101.500 parts by weight of L-carnitine tartrate,
1.000 parts by weight of vitamin B2 (riboflavin),
16.667 parts by weight of bladderwrack extract,
10.000 parts by weight of spirulin,
20.000 parts by weight of Damiana leaf extract,
133.333 parts by weight of Malabar tamarind fruit extract,
4.167 parts by weight of African mango seed extract,
8.003 parts by weight of chicory root extract,
15.000 parts by weight of black elderberry flower extract,
0.250 parts by weight of ground elder leaves extract,
8.333 parts by weight of Shilajit Mumio extract, and
3.333 parts by weight of ash tree leaves extract.
